# EUROPEAN PATENT APPLICATION

(11) **EP 4 589 001 A1**
(43) Date of publication of application: **23.07.2025**
(21) Application number: 23912402.7
(22) Date of filing: 07.04.2023
(51) Int. Cl.: C12N 1/20, C12N 15/01, C12N 13/00, C12P 13/06, C12R 1/15

(54) **MICROORGANISMS WITH ENHANCED L-ALANINE PRODUCTIVITY AND METHOD FOR PRODUCING L-ALANINE USING SAME**

(30) Priority: 30.12.2022 KR 20220190975
(71) Applicant: CJ CheilJedang Corporation, Seoul 04560 (KR)
(72) Inventor: LEE, Ji Hye, Seoul 04560 (KR); KIM, Ju-Yeon, Seoul 04560 (KR); KIM, Min-Jung, Seoul 04560 (KR); CHOI, Jin-Geun, Seoul 04560 (KR); PARK, Jang Hee, Seoul 04560 (KR)
(74) Representative: Meissner Bolte Partnerschaft mbB
(86) International application number: PCT/KR2023/004734
(87) International publication number: WO 2024/143713

(57) **Abstract**

The present invention relates to a mutant strain of *Corynebacterium,* a method for producing the strain, and a method for producing L-alanine using the strain, the mutant strain having improved L-alanine productivity due to radiation-induced mutations compared to the parent strain. Said mutant strain of *Corynebacterium* has improved L-alanine productivity compared to the parent strain, and, as such, use of the strain allows highly efficient and high-yield production of L-alanine.

## Description

### [TECHNICAL FIELD]

The present invention relates to microorganisms with enhanced L-alanine productivity and a method for producing L-alanine using the same.

### [BACKGROUND ART]

L-alanine is an important amino acid that has been broadly applied in the fields of chemistry, food, medicine and the like. It is an amino acid making a sweet taste, and it is applied in the food industry particularly as a flavor enhancer and a nutritional supplement. As of 2020, the global alanine market produces approximately 500 tons per year, and is estimated as over USD 250 million (The Expresswire, 2020; Wendisch, 2014).

In general, the method for production of L-alanine is obtained by chemical synthesis or enzymatic conversion. Chemical synthesis is achieved by modifying Strecker synthesis or Bucherer-Bergs method (Legnani et al., 2021), and enzymatic conversion is obtained by ammonium fumarate from L-aspartic acid (Takamatsu et al., 1982). Most industrial production is achieved through an enzymatic conversion process, and it has problems in that L-aspartic acid, which is a substrate, is produced from petroleum, and production of fumarate also depends on petroleum, so it requires a lot of cost, and when replacing it, the yield is lowered. Therefore, fermentation by microorganisms suggests an interesting alternative by supplying an abundant carbon source at a low cost. Naturally, microorganisms produce L-alanine using aminotransferase or alanine dehydrogenase. However, there are limitations caused by low productivity in producing L-alanine using a naturally derived strain.

Under this background, the present inventors have exerted efforts to develop a microorganism producing L-alanine with higher yield than conventional strains, and as a result, they have found the fact that a mutant strain obtained using *Corynebacterium* produces L-alanine with high yield, thereby completing the present invention.

### [DISCLOSURE]

### [TECHNICAL PROBLEM]

One embodiment of the present invention provides a *Corynebacterium* sp. mutant strain with enhanced L-alanine productivity compared to a parent strain, which is a mutant strain obtained by mutating a parent strain, *Corynebacterium glutamicum* ATCC13869 strain.

Another embodiment of the present invention provides a method for preparing of a *Corynebacterium* sp. mutant strain with enhanced L-alanine productivity compared to a parent strain, comprising
1) mutating by irradiating radiation to a parent strain, *Corynebacterium glutamicum* ATCC13869; and
2) selecting the mutant strain with enhanced L-alanine productivity by culturing the mutated strain of the step 1).

Other embodiment of the present invention provides a composition for producing L-alanine comprising the microorganism with enhanced L-alanine productivity.

Other embodiment of the present invention provides a method for producing L-alanine, comprising culturing the microorganism with enhanced L-alanine productivity in a medium.

Other embodiment of the present invention provides a use of the microorganism with enhanced L-alanine productivity for the production of L-alanine.

### [TECHNICAL SOLUTION]

To specifically describe this is as follows. On the other hand, each description and embodiment disclosed in the present application may also be applied to each other description and embodiment. In other words, all combinations of various elements disclosed in the present application are within the scope of the present application. In addition, the scope of the present application is not to be construed as being limited by the detailed description described below. Furthermore, those skilled in the art can recognize or confirm many equivalents to specific aspects disclosed in the present application using only a common experiment. Moreover, these equivalents are intended to be included in the present application.

The present invention provides a microorganism with enhanced L-alanine productivity.

In the present invention, screening to discover a mutant strain which high-produces L-alanine. As a result, it has been confirmed that 5 kinds of mutant strains prepared by irradiating radiation into the *Corynebacterium glutamicum* ATCC13869 strain has enhanced L-alanine productivity compared to the ATCC13869 strain.

The present invention provides a *Corynebacterium* sp. mutant strain with enhanced L-alanine productivity compared to a parent strain, which is a mutant strain obtained by mutating a parent strain, *Corynebacterium glutamicum* ATCC13869 strain.

The microorganism may be a *Corynebacterium glutamicum* mutant strain.

The *Corynebacterium glutamicum* mutant strain may be any one strain selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).

In the present description, the term, "L-alanine" means an L-amino acid with a chemical formula of "C₃H₇NO₂" which is one of essential amino acids.

In the present description, the term, "productivity" means an ability capable of producing L-alanine, and may be interchangeably used with "production", and may be confirmed by measuring a concentration of L-alanine comprised in a cultured solution after culturing a strain producing L-alanine.

The method for mutating may be performed by various means known in the art, and one method of physical or chemical mutagenesis methods may be used. For example, as a physical mutating method suitable for the present invention, a method of irradiating gamma rays or ultraviolet rays may be used, but not limited thereto. In addition, as a chemical mutating method, N-methyl-N'-nitro-N-nitrosoguanidine (NTG), diepoxibutane, ethylmethane sulfonate, mustard compound, hydrazine and nitrite may be used, but not limited thereto.

The mutating to produce the mutant strain may be by a method of irradiating radiation.

The radiation to induce mutation may be gamma rays or ultraviolet rays, and preferably, it may be gamma rays.

The radiation may be at a dose of 3.0 kGy to 5.0 kGy, 3.1 kGy to 4.9 kGy, 3.2 kGy to 4.8 kGy, 3.3 kGy to 4.7 kGy, 3.4 kGy to 4.6 kGy, 3.5 kGy to 4.5 kGy, 3.6 kGy to 4.4 kGy, 3.7 kGy to 4.3 kGy, 3.8 kGy to 4.2 kGy, 3.9 kGy to 4.1 kGy, preferably, 4.0 kGy per hour, but not limited thereto.

The mutant strain may produce 1.5 times or more L-alanine, and may produce 1.6 times or more, and may produce 1.7 times or more, and may produce 1.5 to 3 times, compared to the parent strain,
The mutant strain may have L-alanine productivity enhanced by 1.5 times or more, enhanced by 1.6 times or more, enhanced by 1.7 times or more, and enhanced by 1.5 times to 3 times, compared to the parent strain.

The mutant strain may have L-alanine productivity increased by 50% or more, 60% or more, 70% or more, 75% or more, 50% to 100%, 60% to 100%, 70% to 100%, 50% to 80%, 60% to 80%, 70% to 80%, or 75%, compared to the parent strain.

The parent strain may be *Corynebacterium glutamicum* ATCC13869, but not limited thereto.

In addition, the present invention provides a method for production of a *Corynebacterium* sp. mutant strain with enhanced L-alanine productivity compared to a parent strain, comprising
1) mutating by irradiating radiation to a parent strain, *Corynebacterium glutamicum* ATCC13869; and
2) selecting the mutant strain with enhanced L-alanine productivity by culturing the mutated strain of the step 1).

The radiation to induce mutation may be gamma rays or ultraviolet rays, and preferably, it may be gamma rays.

The radiation may be at a dose of 3.0 kGy to 5.0 kGy, 3.1 kGy to 4.9 kGy, 3.2 kGy to 4.8 kGy, 3.3 kGy to 4.7 kGy, 3.4 kGy to 4.6 kGy, 3.5 kGy to 4.5 kGy, 3.6 kGy to 4.4 kGy, 3.7 kGy to 4.3 kGy, 3.8 kGy to 4.2 kGy, 3.9 kGy to 4.1 kGy, preferably, 4.0 kGy per hour, but not limited thereto.

Upon mutagenesis, the parent strain is affected by mutagens under a condition sufficient to leave a surviving population of a certain size. The condition varies depending on the type of mutagens, and depends on the amount of mutation that the mutagens induce in the surviving population at a constant death rate (killing rate). For example, in case of gamma rays, the death rate may leave approximately 0.00001% to 20% of the starting subject group, and in case of NTG, the killing rate may leave approximately 10% to 50% of the starting population, and mutation generation by nitrite may leave 0.01% to 0.1% of the starting subject group, but not limited thereto.

In addition, the present invention provides a composition for producing L-alanine comprising a *Corynebacterium* sp. mutant strain with enhanced L-alanine productivity compared to a parent strain, which is a mutant strain obtained by mutating a parent strain, *Corynebacterium glutamicum* ATCC13869 strain.

The microorganism may be a *Corynebacterium glutamicum* mutant strain.

The *Corynebacterium glutamicum* mutant strain may be any one strain selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).

In one example of the present invention, it has been confirmed that the mutant strains obtained by mutating a parent strain, *Corynebacterium glutamicum* ATCC13869, *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P), produce L-alanine at a high concentration. Thus, using a composition comprising the mutant strains, L-alanine can be produced at a high concentration.

Furthermore, the present invention provides a method for producing L-alanine comprising culturing a mutant strain obtained by mutating a parent strain, *Corynebacterium glutamicum* ATCC13869, which is a *Corynebacterium* sp. mutant strain with enhanced L-alanine productivity compared to the parent strain, in a medium.

The method, may further comprise recovering L-alanine from the cultured microorganism, culture or both of them, after the culturing.

In the present description, the term, "culturing" means growing a microorganism in an appropriately artificially controlled environmental condition. The method for culturing a microorganism of the present invention may be performed using a culturing method of *Corynebacterium glutamicum* widely known in the art. Specifically, the example of the culturing method includes batch culture, continuous culture and fed-batch culture, but not limited thereto. These various methods are disclosed, for example, in "Biochemical Engineering" (James M. Lee, Prentice-Hall International Editions, pp138-176, 1991) and the like.

The term of the present invention, "culture" means a material comprising a medium in which a microorganism is growing or grown under an appropriately artificially controlled environmental condition. In a narrow sense, the culture does not include grown microorganisms, but they may be included in a broad sense. The "culture" includes various substances discharged into a medium during growing by a microorganism with medium components composed for culturing of the microorganism, and specifically, it includes alanine as a target substance.

The medium used for culturing should meet requirements of a specific strain in an appropriate manner. Culture media for *Corynebacterium* sp. strains are known. For example, the microorganism of the present application may be cultured while adjusting temperature, pH and the like under an aerobic condition in a conventional medium containing appropriate carbon sources, nitrogen sources, amino acids, vitamins and the like. Then, the carbon sources include carbohydrates such as glucose, fructose and sucrose, amino acids such as glutamic acid and cysteine, and the like. Specifically, natural organic nutrients such as starch hydrolysates, molasses and the like may be used, and preferably, they are carbohydrates such as glucose, fructose, sterilized pre-treated molasses (i.e., molasses converted to reducing sugar) and the like, and other appropriate amounts of carbon sources may be used in various ways without limitation, but not limited thereto. As the nitrogen sources, inorganic nitrogen sources such as ammonia; and organic nitrogen sources such as amino acids such as glutamic acid and cysteine and peptone, meat extract, yeast extract and the like may be used. These nitrogen sources may be used alone or in combination, but not limited thereto. In the medium, as a phosphorus source, phosphoric acid, potassium dihydrogen phosphate or dipotassium hydrogen phosphate or a corresponding sodium-containing salt may be used, but not limited thereto. As the inorganic compound, magnesium sulfate, iron sulfate, manganese sulfate and calcium chloride and the like may be used, and other amino acids, vitamins and appropriate precursors may be included. These media or precursors may be added to the culture in a batch or continuous manner, but not limited thereto.

During culturing, the pH of the culture may be adjusted by adding a compound such as potassium hydroxide, ammonia and phosphate in an appropriate manner. In addition, during culturing, bubble formation may be inhibited by using an antifoaming agent such as fatty acid polyglycol ester. Moreover, in order to maintain the aerobic state of the culture, oxygen or oxygen-containing gas may be injected into the culture. The temperature of the culture is 27°C to 37°C, specifically, 30°C to 33°C. The culturing period may be continued until a production amount of a desired useful substance is obtained, and is specifically 20 to 120 hours.

The recovering L-alanine may be collecting targeted L-alanine from a medium, cultured solution, or microorganism using an appropriate method known in the art according to the culturing method. For example, the recovering may be performed by at least one method selected from centrifugation, filtration, anion exchange chromatography, crystallization, HPLC and the like, but not limited thereto. The method for producing L-alanine, may further comprise purifying, before, at the same time as, or after the recovering.

### [ADVANTAGEOUS EFFECTS]

The *Corynebacterium* sp. mutant strain is a microorganism with enhanced L-alanine productivity compared to a parent strain by mutagenesis. Thus, according to the method for producing L-alanine of the present invention using the *Corynebacterium* sp. mutant strain, L-alanine can be produced with high efficiency and high yield.

### [MODE FOR INVENTION]

Hereinafter, the present invention will be described in more detail by the following examples. However, these examples are intended to illustrate the present invention only, but the scope of the present invention is not limited by these examples.

### Example 1. Selection of mutant strain through artificial mutation method

### 1-1. Establishment of gamma ray irradiation condition to obtain mutant strains with enhanced L-alanine productivity

In order to obtain a microorganism mutant strain with enhanced L-alanine productivity, microorganism mutation was induced using the method as follows.

A physical method, gamma ray irradiation was applied for mutagenesis. High-energy gamma rays emitted from a ray source, Co-60, flow along chromosomes and randomly provoke changes in base sequences such as base substitution, deletion, insertion and the like. As the level of changes in base sequences is proportional to the intensity of irradiation of gamma rays, high-level gamma rays induce mutation at a high rate and simultaneously, also increases the death rate (killing rate) of the strain. In order to sufficiently secure a high-quality mutation library with a high diversity of a high mutation rate, establishment of a condition of irradiating gamma rays against a *Corynebacterium* strain, which is an object of irradiation of gamma rays, was preceded.

Using an irradiation device of high-level gamma rays of Nordion located in Korean Atomic Energy Research Institute, Advanced Radiation Technology Institute, gamma rays with an intensity of 0, 0.5, 1, 2, 3, 4, 5, 7.5, 10 kGy/hr, respectively, were irradiated to a seed medium of 30 mL having an absorbance value at 562 nm of 6.09 for 1 hour, and then, the gamma rays-irradiated solution diluted at a dilution ratio of 10⁰, 10⁻¹, 10⁻², 10⁻³, 10⁻⁴ was smeared on an activation medium by 100 *µ*ℓ, respectively, and after culturing a plate medium in which the gamma rays-irradiated solution was smeared in a 30°C stationary incubator for 48 hours, the number of individual colonies produced was counted to calculate the death rate (killing rate) for each irradiation condition. CFU/mL according to the condition of irradiating gamma rays of 0, 0.5, 1, 2, 3, 4, 5, 7.5 10 kGy/hr was 3.6 X 10⁸, 6.9 X 10⁷, 9.8 X 10⁶, 2.7 X 10⁵, 2.1 X 10³, 3.0 X 10¹, 0, 0, 0, respectively, and the death rate calculated therefrom was shown as 0%, 81.00467%, 97.31540%, 99.92589%, 99.99942%, 99.99999%, 100%, 100%, 100%, respectively. Considering that they are all killed under the condition of irradiating gamma rays of 5 kGy/hr intensity and therefore colonies cannot be secured, 4 kGy/hr intensity was established as a condition of irradiating gamma rays to secure a high-quality mutation library with a high diversity of mutation through irradiation of gamma rays based on a *Corynebacterium* strain.

### 1-2. Selection of mutant strains with enhanced L-alanine productivity

In order to obtain a microorganism with high L-alanine productivity, a mutation library based on irradiation of gamma rays was constructed for *Corynebacterium glutamicum* ATCC13869 strain, and variants with high L-alanine productivity were screened. For irradiation of gamma rays against the ATCC13869 strain, a 500 mL flask comprising a seed medium of 50 mL was cultured in a shaking incubator of 30°C for 24 hours to secure a cultured solution having an absorbance value at 562 nm of 7.84, and then, it was diluted using the seed medium so that the absorbance value at 562 nm was 6.10. Using an irradiation device of high-level gamma rays located in Korean Atomic Energy Research Institute, Advanced Radiation Technology Institute, gamma rays of 4 kGy/hr were irradiated to the diluted solution of 30 mL for 1 hour, and then, the stock solution of the irradiation solution was smeared on an activation medium of 100 sheets of 90 X 15 mm petri dishes by dividing it by 100 *µ*ℓ, respectively. After that, the petri dishes were cultured in a 30 °C stationary incubator for 48 hours to secure a total of about 40,000 individual colonies, and to secure the stability of the variants, all the individual colonies produced in the entire petri dishes were recovered and suspended in a 20% glycerol solution, and then, they subdivided into 1.5 mL microtubes by 1 mL each and stored in a cryogenic freezer at -80°C.

During screening of variants with high L-alanine productivity, the 20% glycerol suspension taken out of the cryogenic freezer and thawed at a room temperature was continuously diluted using saline to 10⁻¹, 10⁻², 10⁻³, 10⁻⁴, and 10⁻⁵, and then the diluted solution corresponding to each dilution factor was smeared on an activation medium by 100 *µ*ℓ each, and reactivated in an individual colony form through culturing in a 30°C stationary incubator for 24 hours. The reactivated individual colony was inoculated into 96 Deep Well Plate in which a production medium of 350 *µ*ℓ corresponding to a filling rate of 17& was aliquoted per each well using Qpix420 equipment, Colony Picker equipment of Molecular Devices, and the ATCC13869 strain, to which gamma rays were not irradiated, was inoculated into four wells per plate on the same plate, and it was used as a control group for screening of the variants with high L-alanine productivity.

The 96 Deep Well Plate, in which the control group strain and variants were inoculated, respectively, was sealed using Gas permeable seal mark2 of Azenta, and then was cultured in Multitron equipment, a shaking incubator of Infors-HT under a condition of 30°C, 1,000 rpm for 48 hours. The 96 Deep Well Plate cultured for 48 hours was centrifuged in Centrifuge 5810R equipment, a centrifuge of Eppendorf under a condition of 15°C, 4,000 rpm for 20 minutes, and then for NIR spectrometry analysis, 100 *µ*ℓ culture supernatant from which microbial cells were separated was transferred to 96 Well Black Polystyrene Microplate of Corning using Biomek i5, Liquid handler equipment of Beckman Coulter. Then, by applying NIR Spectrometry self-manufactured in Analysis & Quality department of BIO Technology Research Center of CJ CheilJedang, individual analysis spectra for each well were secured, and a selection logic of 15% or more enhancement in the L-alanine concentration compared to the control group was applied by applying to a regression analysis prediction model with a determination coefficient of 0.96 for the L-alanine 0 ~ 20 g/L concentration section established on the basis of the culture sample in which the L-alanine concentration was quantified through pre-HPLC analysis, and thereby, 64 strains were primarily selected among 10,304 variants. Culturing was carried out for the selected 64 strains by the same method as above to finally select the top 5 kinds with the high L-alanine concentration.

The mutant strains obtained by the above method were named *Corynebacterium glutamicum* CJ0230, CJ2011, CJ2015, CJ2057, and CJ2064, and they were deposited to Korean Culture Center of Microorganisms, which is an international depository authority under the Budapest Treaty, on December 19, 2022, and were given accession numbers KCCM13305P, KCCM13306P, KCCM13307P, KCCM13308P, and KCCM13309P, respectively.

The compositions of the media used in Example 1 and Example 2 are as follows.

### <Activation medium>

Beef extract 5 g/L, polypeptone 10 g/L, yeast extract 5 g/L, urea 2 g/L, sodium chloride (NaCl) 2.5 g/L, agar 20 g/L, glucose 10 g/L, 10N sodium hydroxide (NaOH) 100 *µ*ℓ/L

### <Seed medium>

Glucose (anhydrous glucose) 20 g/L, polypeptone 10 g/L, yeast extract 10 g/L, ammonium sulfate [(NH₄)₂SO₄] 10 g/L, urea 1.5 g/L, potassium phosphate monobasic (KH₂PO₄) 5.2 g/L, potassium phosphate dibasic (K₂HPO₄) 10.7 g/L, d-Biotin 1.8 mg/L, thiamine-HCl 9 mg/L, CAPA 9 mg/L, NCA 60 mg/L, magnesium sulfate (MgSO₄) 0.5 g/L

### <Production medium>

Calcium carbonate (CaCO₃) 30 g/L, sucrose 57 g/L, BM 6 g/L, magnesium sulfate (MgSO₄) 0.5 g/L, (NH₄)₂SO₄ 50 g/L, KH₂PO₄ 1 g/L, yeast extract 2g/L, ammonium acetate 6.28 g/L, d-Biotin 0.05 mg/L, thiamine-HCl 0.1 mg/L, MnSO₄ 6.7 mg/L, FeSO₄ 10 mg/L

### Example 2. Evaluation of L-alanine productivity of L-alanine producing mutant strains

In order to confirm the L-alanine productivity of the *Corynebacterium glutamicum* CJ0230, CJ2011, CJ2015, CJ2057 and CJ2064 strains obtained in Example 1 above, they were cultured by the method as follows.

Specifically, after inoculating the parent strain, *Corynebacterium glutamicum* ATCC13869 strain and 5 kinds of the mutant strains to a 250 ml corner-baffled flask containing a seed medium of 25 ml, respectively, they were cultured with shaking at 200 rpm at 30°C for 20 hours to obtain a seed cultured solution. After that, the seed cultured solution of 1 ml was inoculated to a 250 ml corner-baffled flask containing the production medium of 24 ml, and it was cultured at 200 rpm at 30°C for 72 hours to prepare L-alanine. After completing the culturing, the concentration of L-alanine comprised in the cultured solution using high-performance liquid chromatography (HPLC) to measure L-alanine production of each strain. The obtained result was shown in Table 1 below.

**[Table 1]**

| Comparison of L-alanine productivity of *Corynebacterium glutamicum* CJ0230 strain, CJ2011 strain, CJ2015 strain, CJ2057 strain and CJ2064 strain | | | | | | |
|---|---|---|---|---|---|---|
| | ATCC 13869 (parent strain) | CJ0230 (mutant strain 1) | CJ2011 (mutant strain 2) | CJ2015 (mutant strain 3) | CJ2057 (mutant strain 4) | CJ2064 (mutant strain 5) |
| L-Alanine concentration (g/l) | 6.1 | 10.7 | 10.4 | 9.9 | 10.1 | 10.5 |

As a result, as shown in Table 1 above, the parent strain, *Corynebacterium glutamicum* ATCC13869 strain produced L-alanine at a concentration of 6.1 g/l. The mutant *Corynebacterium glutamicum* CJ0230 strain, CJ2011 strain, CJ2015 strain, CJ2057 strain and CJ2064 strain according to the present invention produced L-alanine at a concentration of 10.7 g/l, 10.4 g/l, 9.9 g/l, 10.1 g/l and 10.5 g/l, respectively, and thereby, it was confirmed that they had L-alanine productivity of about 162% or more at minimum compared to the parent strain.

The above result means that 5 kinds of strains of the *Corynebacterium glutamicum* mutant strains according to the present invention can produce L-alanine with high efficiency and high yield.

### [ACCESSION NUMBER]

Name of depository authority: Korean Culture Center of Microorganisms (KCCM)
Accession number: KCCM13305P
Accession date: 20221219
Name of depository authority: Korean Culture Center of Microorganisms (KCCM)
Accession number: KCCM13306P
Accession date: 20221219
Name of depository authority: Korean Culture Center of Microorganisms (KCCM)
Accession number: KCCM13307P
Accession date: 20221219
Name of depository authority: Korean Culture Center of Microorganisms (KCCM)
Accession number: KCCM13308P
Accession date: 20221219
Name of depository authority: Korean Culture Center of Microorganisms (KCCM)
Accession number: KCCM13309P
Accession date: 20221219

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ0230 | Accession number given by the
INTERNATIONAL DEPOSITARY AUTHORITY:
KCCM13305P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 19. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2022. 한국미생물보존센터장인

¹ Where Rule 6.4(d) applies, such date is the date on which the status of international depositary authority was
acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2011 | Accession number given by the
INTERNATIONAL DEPOSITARY AUTHORITY:
KCCM13306P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 19. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2022 한국미생물보존센터장의인

¹ Where Rule 6.4(d) applies, such date is the date on which the status of international depositary authority was
acquired.

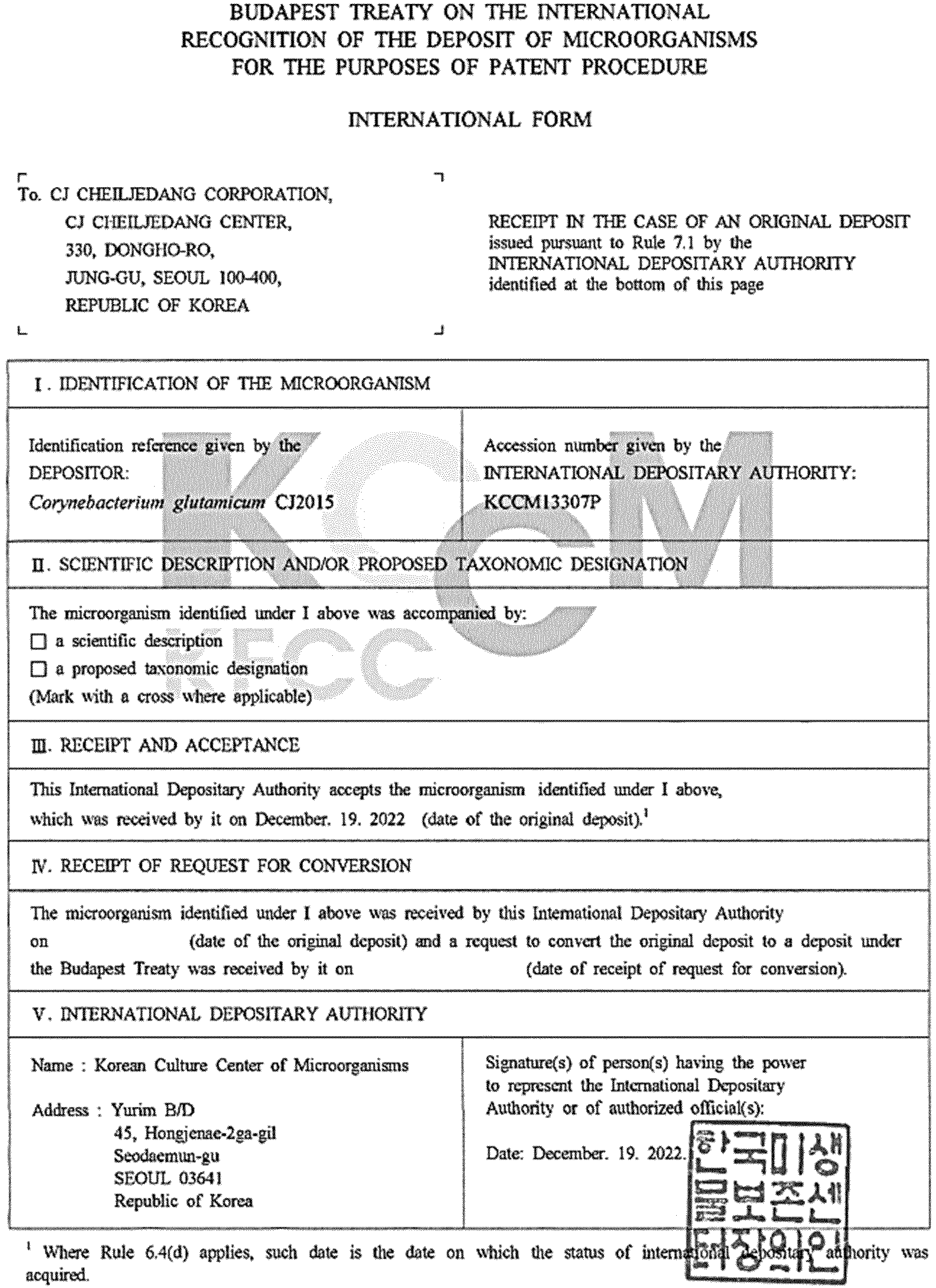
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2015 | Accession number given by the
INTERNATIONAL DEPOSITARY AUTHORITY:
KCCM13307P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 19. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2022.

¹ Where Rule 6.4(d) applies, such date is the date on which the status of international depositary authority was
acquired.

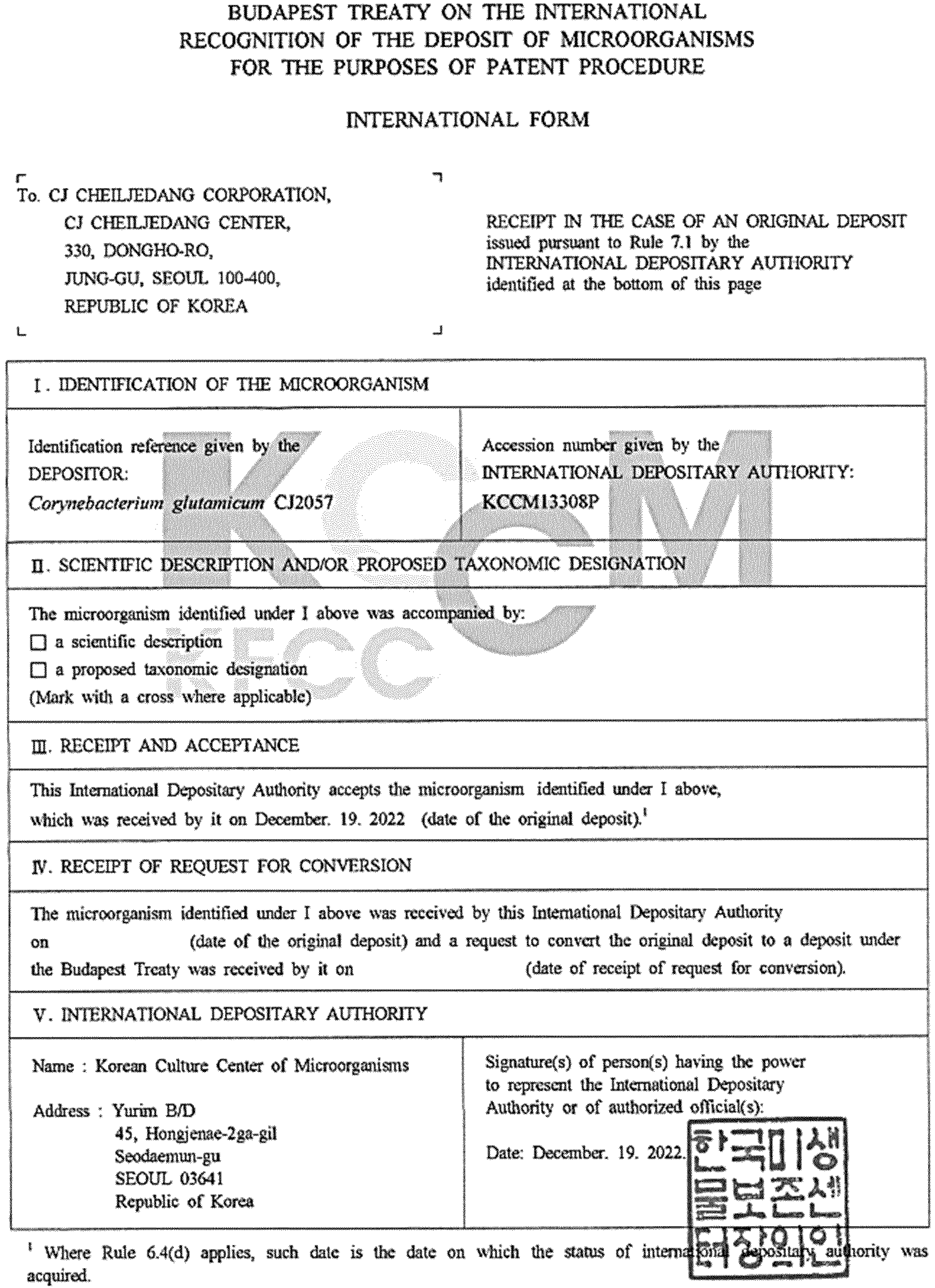
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2057 | Accession number given by the
INTERNATIONAL DEPOSITARY AUTHORITY:
KCCM13308P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on December. 19. 2022 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2022.

¹ Where Rule 6.4(d) applies, such date is the date on which the status of international depositary authority was
acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CJ2064 | Accession number given by the
INTERNATIONAL DEPOSITARY AUTHORITY:
KCCM13309P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 19. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 19. 2022. 한국미생물보존센터장의인

¹ Where Rule 6.4(d) applies, such date is the date on which the status of international depositary authority was
acquired.

## Claims

1. A *Corynebacterium* sp. mutant strain with enhanced L-alanine productivity compared to a parent strain, which is a mutant strain obtained by mutating a parent strain, *Corynebacterium glutamicum* ATCC13869 strain.

2. The *Corynebacterium* sp. mutant strain according to claim 1, wherein the *Corynebacterium* sp. mutant strain is *Corynebacterium glutamicum.*

3. The *Corynebacterium* sp. mutant strain according to claim 1, wherein the *Corynebacterium* sp. mutant strain is any one selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).

4. The *Corynebacterium* sp. mutant strain according to claim 1, wherein the mutating is by a method of irradiating radiation.

5. The *Corynebacterium* sp. mutant strain according to claim 4, wherein the radiation is gamma rays.

6. The *Corynebacterium* sp. mutant strain according to claim 4, wherein the mutating is by irradiating radiation at a dose of 3.5 kGy to 4.5 kGy per hour.

7. The *Corynebacterium* sp. mutant strain according to claim 1, wherein the mutant strain has L-alanine productivity enhanced by 1.5 times or more compared to the parent strain, *Corynebacterium glutamicum* ATCC13869.

8. A method for preparing of a *Corynebacterium* sp. mutant strain with enhanced L-alanine productivity compared to a parent strain, comprising 1) mutating by irradiating radiation to a parent strain, *Corynebacterium glutamicum* ATCC13869; and
2) selecting a mutant strain with enhanced L-alanine productivity by culturing the mutated strain of the step 1).

9. The method for preparing of a *Corynebacterium* sp. mutant strain according to claim 8, wherein the radiation is gamma rays.

10. The method for preparing of a *Corynebacterium* sp. mutant strain according to claim 8, wherein the radiation is irradiated at a dose of 3.5 kGy to 4.5 kGy per hour

11. The method for preparing of a *Corynebacterium* sp. mutant strain according to claim 8, wherein the *Corynebacterium* sp. mutant strain is *Corynebacterium glutamicum.*

12. The method for preparing of a *Corynebacterium* sp. mutant strain according to claim 8, wherein the *Corynebacterium* sp. mutant strain is any one selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).

13. The method for preparing of a *Corynebacterium* sp. mutant strain according to claim 8, wherein the mutant strain has L-alanine productivity enhanced by 1.5 times or more compared to the parent strain, *Corynebacterium glutamicum* ATCC13869.

14. A composition for producing L-alanine comprising the *Corynebacterium* sp. mutant strain of claim 1.

15. The composition for producing L-alanine according to claim 14, wherein the *Corynebacterium* sp. mutant strain is any one selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).

16. A method for producing L-alanine, comprising culturing the *Corynebacterium* sp. mutant strain of claim 1 in a medium.

17. The method for producing L-alanine according to claim 16, wherein the *Corynebacterium* sp. mutant strain is any one selected from the group consisting of *Corynebacterium glutamicum* CJ0230 strain (accession number KCCM13305P), *Corynebacterium glutamicum* CJ2011 strain (accession number KCCM13306P), *Corynebacterium glutamicum* CJ2015 strain (accession number KCCM13307P), *Corynebacterium glutamicum* CJ2057 strain (accession number KCCM13308P) and *Corynebacterium glutamicum* CJ2064 strain (accession number KCCM13309P).
